(19) Europäisches Patentamt
European Patent Office
Office européen des brevets

(11) Veröffentlichungsnummer : **0 006 538**
**B1**

(12) # EUROPÄISCHE PATENTSCHRIFT

(45) Veröffentlichungstag der Patentschrift : **02.12.81**

(51) Int. Cl.[3] : **C 07 D249/08**, A 01 N 43/64

(21) Anmeldenummer : **79101939.1**

(22) Anmeldetag : **15.06.79**

(54) **Sulfenylierte Carbamoyl-triazolyl-O,N-acetale, Verfahren zu ihrer Herstellung und ihre Verwendung als Fungizide.**

(30) Priorität : **26.06.78 DE 2827968**

(43) Veröffentlichungstag der Anmeldung : **09.01.80 (Patentblatt 80/01)**

(45) Bekanntmachung des Hinweises auf die Patenterteilung : **02.12.81 Patentblatt 81/48**

(84) Benannte Vertragsstaaten : **BE CH DE FR GB IT NL**

(56) Entgegenhaltungen :
**DE - A1 - 2 600 799**
**DE - A1 - 2 635 663**
**DE - A1 - 2 635 883**
**DE - A1 - 2 720 949**

(73) Patentinhaber : **BAYER AG**
**Zentralbereich Patente, Marken und Lizenzen**
**D-5090 Leverkusen 1, Bayerwerk (DE)**

(72) Erfinder : **Krämer, Wolfgang, Dr.**
**Am Eckbusch 39/162**
**D-5600 Wuppertal 1 (DE)**
Erfinder : **Büchel, Karl-Heinz, Prof. Dr.**
**Bergerheide 62**
**D-5600 Wuppertal (DE)**
Erfinder : **Kühle, Engelbert, Dr.**
**von Bodelschwingh Strasse 42**
**D-5060 Bergisch-Gladbach 2 (DE)**
Erfinder : **Frohberger, Paul-Ernst, Dr.**
**Willi-Baumeister-Strasse 5**
**D-5090 Leverkusen (DE)**
Erfinder : **Brandes, Wilhelm, Dr.**
**Eichendorffstrasse 3**
**D-5653 Leichlingen (DE)**

**EP 0 006 538 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Sulfenylierte Carbamoyl-triazolyl-O,N-acetale, Verfahren zu ihrer Herstellung und ihre Verwendung als Fungizide

Die vorliegende Erfindung betrifft neue sulfenylierte Carbamoyl-triazolyl-O,N-acetale, ein Verfahren zu ihrer Herstellung und ihre Verwendung als Fungizide.

Es ist bereits bekannt geworden, daß Trityl-triazole, wie Triphenyl-(1,2,4-triazol-1-yl)-methan, eine gute fungizide Wirksamkeit besitzen (vgl. DE-A 1 795 249). Außerdem ist bereits bekannt geworden, daß acylierte Triazolyl-O,N-acetale, wie insbesondere im Phenylteil substituierte 2-Acyloxy-3,3-dimethyl-1-phenoxy-1-(1,2,4-triazol-1-yl)-butane, gute fungizide Eigenschaften aufweisen (vgl. DE-A-2 600 799). Die Wirkung dieser Verbindungen ist jedoch, insbesondere bei niedrigen Auswandmengen und -konzentrationen, nicht immer ganz befriedigend.

Es wurden neue sulfenylierte Carbamoyl-triazolyl-O,N-acetale der allgemeinen Formel

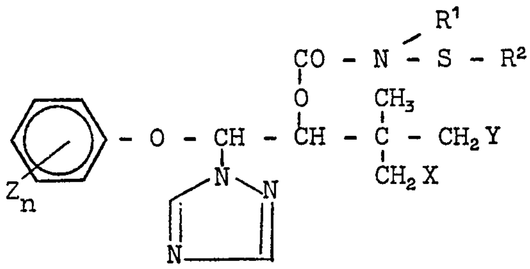

(I)

in welcher

$R^1$ für Methyl oder gegebenenfalls durch Chlor oder Methyl substituiertes Phenyl steht,

$R^2$ für Trifluormethyl, Chlordifluormethyl, Dichlorfluormethyl und Trichlormethyl steht,

X und Y für Wasserstoff, Fluor, Chlor oder Brom stehen,

Z für Fluor, Chlor, Brom, Methyl, Äthyl, sowie für gegebenenfalls durch Chlor oder Methyl substituiertes Phenyl steht, und

n für die Zahlen 0, 1 oder 2 steht,

und deren physiologisch verträglichen Säureadditions-Salze und Metallsalz-Komplexe gefunden. Sie weisen starke fungizide Eigenschaften auf.

Die Verbindungen der Formel (I) besitzen zwei asymmetrische Kohlenstoffatome ; sie können deshalb in der erythrowie in der threo-Form vorliegen. In beiden Fällen liegen sie vorwiegend als Racemate vor.

Man erhält die sulfenylierten Carbamoyl-triazolyl-O,N-acetale der Formel (I), indem man ein Alkoholat eines 2-Hydroxy-1-phenoxy-1-triazolyl-butans der Formel

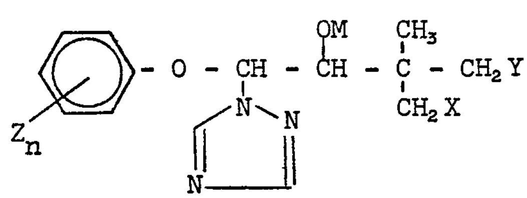

(II)

in welcher

X, Y, Z und n die oben angegebene Bedeutung haben und

M für ein Alkalimetall, eine quarternäre Ammonium- oder Phosphonium-Gruppe steht,

mit einem sulfenylierten Carbamoylfluorid der Formel

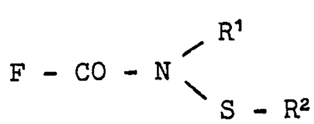

(III)

in welcher

$R^1$ und $R^2$ die oben angegebene Bedeutung haben,

in Gegenwart eines Verdünnungsmittels umsetzt.

Weiterhin können die erfindungsgemäß erhältlichen sulfenylierten Carbamoyl-triazolyl-O,N-acetale der Formel (I) durch Umsetzung mit Säuren in die Salze überführt werden, bzw. können durch Reaktion mit Metallsalzen die entsprechenden Metallsalz-Komplexe erhalten werden.

Ueberraschenderweise zeigen die erfindungsgemäßen sulfenylierten Carbamoyl-triazolyl-O,N-acetale eine erheblich höhere fungizide Wirksamkeit, insbesondere gegen Rost- und Mehltauarten, als die aus dem Stand der Technik bekannten acylierten Triazolyl-O,N-acetale, welche chemisch und wirkungsmäßig naheliegende Verbindungen sind, und als das Triphenyl-(1,2,4-triazol-1-yl)-methan, welches eine Verbindung gleicher Wirkungsrichtung ist. Die erfindungsgemäßen Stoffe stellen somit eine Bereicherung

der Technik dar.

Im Einzelnen seien außer den bei den Herstellungsbeispielen aufgelisteten Verbindungen noch die folgenden Verbindungen der allgemeinen Formel (I)

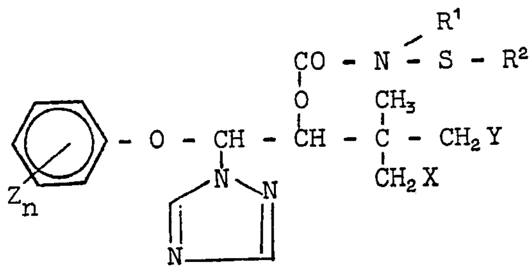

(I)

mit den folgenden Substituentenbedeutungen genannt :

| $R^1$ | $R^2$ | X | Y | $Z_n$ |
|---|---|---|---|---|
| $CH_3$ | $CCl_3$ | H | H | — |
| $CH_3$ | $CCl_3$ | H | H | 4—Br |
| $CH_3$ | $CCl_3$ | H | H | 4—F |
| $CH_3$ | $CCl_3$ | H | H | 2,4—$Cl_2$ |
| $CH_3$ | $CCl_3$ | H | H | 2—$CH_3$, 4-Cl |
| $CH_3$ | $CCl_3$ | H | H | 2—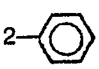 |
| 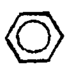 | $CCl_3$ | H | H | — |
| 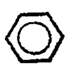 | $CCl_3$ | H | H | 4—Cl |
| 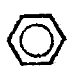 | $CCl_3$ | H | H | 4—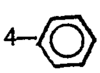 |
| 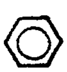 | $CCl_3$ | H | H | 2—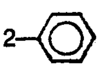 |
| 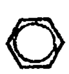 | $CCl_3$ | H | H | 4—Br |
| 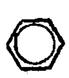 | $CCl_3$ | H | H | 4—F |
| 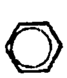 | $CCl_3$ | H | H | 2,4—$Cl_2$ |
| 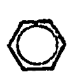 | $CCl_3$ | H | H | 2—$CH_3$, 4—Cl |
| $CH_3$ | $CCl_2F$ | H | H | — |

| $R^1$ | $R^2$ | X | Y | $Z_n$ |
|---|---|---|---|---|
| $CH_3$ | $CCl_2F$ | H | H | 4—Br |
| $CH_3$ | $CCl_2F$ | H | H | 4—F |
| $CH_3$ | $CCl_2F$ | H | H | 2,4—$Cl_2$ |
| $CH_3$ | $CCl_2F$ | H | H | 2—$CH_3$, 4—Cl |
| $CH_3$ | $CCl_2F$ | H | H | 2—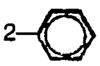 |
| 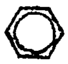 | $CCl_2F$ | H | H | — |
| 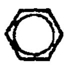 | $CCl_2F$ | H | H | 4—Cl |
| 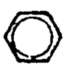 | $CCl_2F$ | H | H | 4—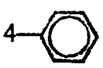 |
| 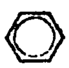 | $CCl_2F$ | H | H | 2—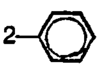 |
| 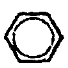 | $CCl_2F$ | H | H | 4—Br |
| 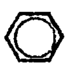 | $CCl_2F$ | H | H | 4—F |
| 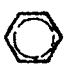 | $CCl_2F$ | H | H | 2,4—$Cl_2$ |
| 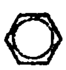 | $CCl_2F$ | H | H | 2—$CH_3$, 4—Cl |
| $CH_3$ | $CCl_3$ | Cl | H | — |
| $CH_3$ | $CCl_3$ | Cl | H | 4—Cl |
| $CH_3$ | $CCl_3$ | Cl | H | 4—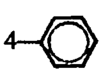 |
| $CH_3$ | $CCl_3$ | Cl | H | 2—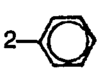 |
| $CH_3$ | $CCl_3$ | Cl | H | 4—Br |
| $CH_3$ | $CCl_3$ | Cl | H | 4—F |
| $CH_3$ | $CCl_3$ | Cl | H | 2,4—$Cl_2$ |
| $CH_3$ | $CCl_3$ | Cl | H | 2—$CH_3$, 4—Cl |

| $R^1$ | $R^2$ | X | Y | $Z_n$ |
|---|---|---|---|---|
| 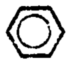 | $CCl_3$ | Cl | H | — |
| 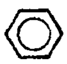 | $CCl_3$ | Cl | H | 4—Cl |
| 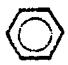 | $CCl_3$ | Cl | H | 4— 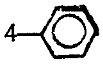 |
| 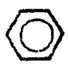 | $CCl_3$ | Cl | H | 2— 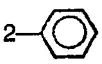 |
| 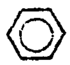 | $CCl_3$ | Cl | H | 4—Br |
| 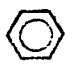 | $CCl_3$ | Cl | H | 4—F |
| 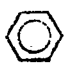 | $CCl_3$ | Cl | H | 2,4—$Cl_2$ |
| 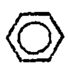 | $CCl_3$ | Cl | H | 2—$CH_3$, 4—Cl |
| $CH_3$ | $CCl_2F$ | Cl | H | — |
| $CH_3$ | $CCl_2F$ | Cl | H | 4—Cl |
| $CH_3$ | $CCl_2F$ | Cl | H | 4— 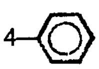 |
| $CH_3$ | $CCl_2F$ | Cl | H | 2— 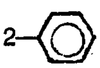 |
| $CH_3$ | $CCl_2F$ | Cl | H | 4—Br |
| $CH_3$ | $CCl_2F$ | Cl | H | 4—F |
| $CH_3$ | $CCl_2F$ | Cl | H | 2,4—$Cl_2$ |
| $CH_3$ | $CCl_2F$ | Cl | H | 2—$CH_3$, 4—Cl |
| 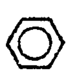 | $CCl_2F$ | Cl | H | — |
| 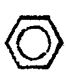 | $CCl_2F$ | Cl | H | 4—Cl |
| 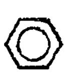 | $CCl_2F$ | Cl | H | 2— 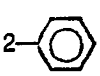 |
| 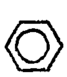 | $CCl_2F$ | Cl | H | 4—Br |
| 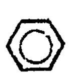 | $CCl_2F$ | Cl | H | 4—F |

| $R^1$ | $R^2$ | X | Y | $Z_n$ |
|---|---|---|---|---|
| 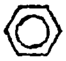 | $CCl_2F$ | Cl | H | 2,4—$Cl_2$ |
| 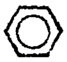 | $CCl_2F$ | Cl | H | 2—$CH_3$, 4—Cl |
| 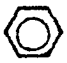 | $CCL_2F$ | Cl | H | 4—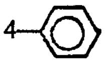 |
| $CH_3$ | $CCl_3$ | Cl | Cl | 4—Cl |
| $CH_3$ | $CCl_3$ | Cl | Cl | 4—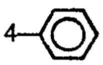 |
| $CH_3$ | $CCl_2$ | Cl | Cl | 2—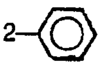 |
| $CH_3$ | $CCl_3$ | Cl | Cl | — |
| $CH_3$ | $CCl_3$ | Cl | Cl | 4—Br |
| $CH_3$ | $CCl_3$ | Cl | Cl | 4—F |
| $CH_3$ | $CCl_3$ | Cl | Cl | 2,4—$Cl_2$ |
| $CH_3$ | $CCl_3$ | Cl | Cl | 2—$CH_3$, 4—Cl |
| $CH_3$ | $CCl_2F$ | Cl | Cl | — |
| $CH_3$ | $CCl_2F$ | Cl | Cl | 4—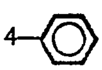 |
| $CH_3$ | $CCl_2F$ | Cl | Cl | 2—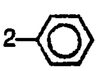 |
| $CH_3$ | $CCl_2F$ | Cl | Cl | 4—Br |
| $CH_3$ | $CCl_2F$ | Cl | Cl | 4—F |
| $CH_3$ | $CCl_2F$ | Cl | Cl | 2,4—$Cl_2$ |
| $CH_3$ | $CCl_2F$ | Cl | Cl | 2—$CH_3$, 4—Cl |
| $CH_3$ | $CCl_3$ | Br | H | — |
| $CH_3$ | $CCl_3$ | Br | H | 4—Cl |
| $CH_3$ | $CCl_3$ | Br | H | 4—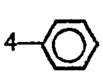 |

| $R^1$ | $R^2$ | X | Y | $Z_n$ |
|---|---|---|---|---|
| $CH_3$ | $CCl_3$ | Br | H | 2— 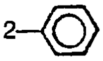 |
| $CH_3$ | $CCl_3$ | Br | H | 4—Br |
| $CH_3$ | $CCl_3$ | Br | H | 4—F |
| $CH_3$ | $CCl_3$ | Br | H | 2,4—$Cl_2$ |
| $CH_3$ | $CCl_3$ | Br | H | 2—$CH_3$, 4—Cl |

Verwendet man beispielsweise das Natriumalkanolat des 1-(4-Chlorphenoxy)-3,3-dimethyl-1-(1,2,4-triazol-1-yl)-butan-2-ols und N-Methyl-N-trichlormethylmercaptocarbamoylfluorid als Ausgangsstoffe, so kann der Reaktionsablauf durch das folgende Formelschema wiedergegeben werden :

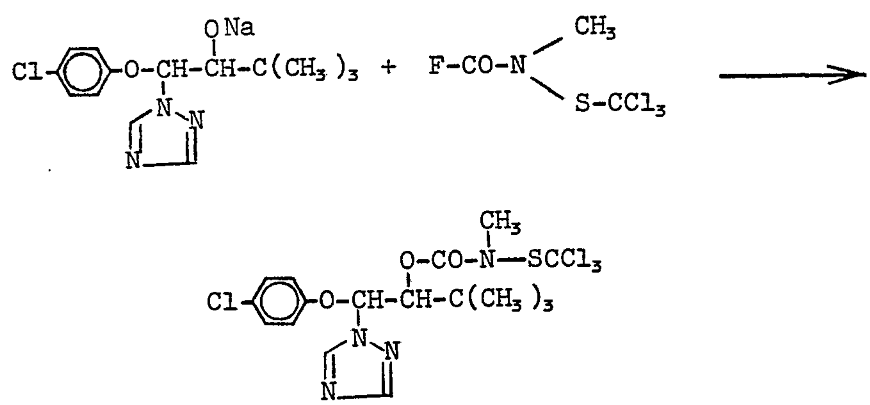

Die als Ausgangsstoffe zu verwendenden Alkoholate von 2-Hydroxy-1-phenoxy-1-triazolyl-butanen sind durch die Formel (II) allgemein definiert. In dieser Formel steht *M* vorzugsweise für die Alkalimetalle Lithium, Natrium und Kalium. *M* steht außerdem vorzugsweise für folgende quarternäre Ammoniumgruppen : Tetrabutylammonium, N-Benzyl-N,N,N-trimethylammonium, Hexadecyl-trimethyl-ammonium, 2-Hydroxyäthyl-trimethyl-ammonium, Tetraäthyl-ammonium, Tetramethyl-ammonium, Tetra-n-propylammonium, (Cyclopropylmethyl)-trimethyl-ammonium, Methyl-trioctyl-ammonium, N-Phenyl-N,N,N-trimethyl-ammonium, N-(4-Methylbenzyl)-N,N,N-trimethyl-ammonium, N-Benzyl-N,N-dimethyl-N-dodecyl-ammonium, N,N-Dibenzyl-N,N-dimethyl-ammonium, Benzyl-dimethyl-n-hexadecyl-ammonium, Benzyldimethyl-tetradecyl-ammonium, Benzyl-tributylammonium, Benzyl-triäthyl-ammonium, Butyl-tripropylammonium, Octadecyl-trimethyl-ammonium, Tetrahexyl-ammonium, Tetra-octyl-ammonium, Tetrapentyl-ammonium, Tricaprylmethyl-ammonium und Hexadecylpyridinium sowie vorzugsweise für folgende Phosphoniumgruppen : Tetraphenylphosphonium, Hexadecyltributyl-phosphonium, Aethyltriphenyl-phosphonium oder Methyl-triphenyl-phosphonium.

Die Alkoholate der Formel (II) sind noch nicht bekannt. Man erhält sie, indem man die entsprechenden 2-Hydroxy-1-phenoxy-1-triazolyl-butane mit geeigneten starken Basen, wie Alkalimetallamiden oder -hydriden, quaternären Ammoniumhydroxiden oder Phosphoniumhydroxiden in einem indifferenten Lösungsmittel umsetzt. Die genannten 2-Hydroxy-1-phenoxy-1-triazolyl-butane sind bekannt (vgl. DE-A-2 324 010 und DE-A-2 632 603).

Als Beispiele für die 2-Hydroxy-1-phenoxy-1-triazolyl-butane, die den erfindungsgemäß als Ausgangsstoffe zu verwendenden Alkoholaten der Formel (II) zugrunde liegen, seien genannt :

2-Hydroxy-3,3-dimethyl-1-phenoxy-1-(1,2,4-triazol-1-yl)-butan
2-Hydroxy-3,3-dimethyl-1-(4-chlorphenoxy)-1-(1,2,4-triazol-1-yl)-butan
2-Hydroxy-3,3-dimethyl-1-(2,4-dichlorphenoxy)-1-(1,2,4-triazol-1-yl)-butan

2-Hydroxy-3,3-dimethyl-1-(4-bromphenoxy)-1-(1,2,4-triazol-1-yl)-butan
2-Hydroxy-3,3-dimethyl-1-(4-biphenylyloxy)-1-(1,2,4-triazol-1-yl)-butan
2-Hydroxy-3,3-dimethyl-1-(2-biphenylyloxy)-1-(1,2,4-triazol-1-yl)-butan
2-Hydroxy-3,3-dimethyl-1-(4'-chlor-4-biphenylyloxy)-1-(1,2,4-triazol-1-yl)-butan
2-Hydroxy-3,3-dimethyl-1-(4-bromphenoxy)-1-(1,2,4-triazol-1-yl)-butan
2-Hydroxy-3,3-dimethyl-1-(4-fluorphenoxy)-1-(1,2,4-triazol-1-yl)-butan
2-Hydroxy-3,3-dimethyl-(4-chlor-2-methylphenoxy)-1-(1,2,4-triazol-1-yl)-butan
4-Chlor-2-hydroxy-3,3-dimethyl-1-phenoxy-1-(1,2,4-triazol-1-yl)-butan
4-Chlor-2-hydroxy-3,3-dimethyl-1-(4-chlorphenoxy)-1-(1,2,4-triazol-1-yl)-butan
4-Chlor-2-hydroxy-3,3-dimethyl-1-(2,4-dichlorphenoxy)-1-(1,2,4-triazol-1-yl)-butan
4-Chlor-2-hydroxy-3,3-dimethyl-1-(4-bromphenoxy)-1-(1,2,4-triazol-1-yl)-butan
4-Chlor-2-hydroxy-3,3-dimethyl-1-(4-biphenylyloxy)-1-(1,2,4-triazol-1-yl)-butan
4-Chlor-2-hydroxy-3,3-dimethyl-1-(2-biphenylyloxy)-1-(1,2,4-triazol-1-yl)-butan
4-Chlor-2-hydroxy-3,3-dimethyl-1-(4'-chlor-4-biphenylyloxy)-1-(1,2,4-triazol-1-yl)-butan
4-Chlor-2-hydroxy-3,3-dimethyl-1-(4-fluorphenoxy)-1-(1,2,4-triazol-1-yl)-butan
4-Chlor-2-hydroxy-3,3-dimethyl-1-(4-chlor-2-methylphenoxy)-1-(1,2,4-triazol-1-yl)-butan
4-Brom-2-hydroxy-3,3-dimethyl-1-phenoxy-1-(1,2,4-triazol-1-yl)-butan
4-Brom-2-hydroxy-3,3-dimethyl-1-(4-chlorphenoxy)-1-(1,2,4-triazol-1-yl)-butan
4-Brom-2-hydroxy-3,3-dimethyl-1-(2,4-dichlorphenoxy)-1-(1,2,4-triazol-1-yl)-butan
4-Brom-2-hydroxy-3,3-dimethyl-1-(4-bromphenoxy)-1-(1,2,4-triazol-1-yl)-butan
4-Brom-2-hydroxy-3,3-dimethyl-1-(4-phenoxyphenoxy)-1-(1,2,4-triazol-1-yl)-butan
4-Brom-2-hydroxy-3,3-dimethyl-1-(4'-chlor-4-phenoxyphenoxy)-1-(1,2,4-triazol-1-yl)-butan
4-Brom-2-hydroxy-3,3-dimethyl-1-(4-fluorphenoxy)-1-(1,2,4-triazol-1-yl)-butan
4-Brom-2-hydroxy-3,3-dimethyl-1-(4-fluorphenoxy)-1-(1,2,4-triazol-1-yl)-butan
4-Brom-2-hydroxy-3,3-dimethyl-1-(4-chlor-2-methyl-phenoxy)-1-(1,2,4-triazol-1-yl)-butan
4-Chlor-3-chlormethyl-2-hydroxy-3-methyl-1-phenoxy-1-(1,2,4-triazol-1-yl)-butan
4-Chlor-3-chlormethyl-2-hydroxy-3-methyl-1-(4-chlorphenoxy)-1-(1,2,4-triazol-1-yl)-butan
4-Chlor-3-chlormethyl-2-hydroxy-3-methyl-1-(2,4-dichlorphenoxy)-1-(1,2,4-triazol-1-yl)-butan
4-Chlor-3-chlormethyl-2-hydroxy-3-methyl-1-(4-bromphenoxy)-1-(1,2,4-triazol-1-yl)-butan
4-Chlor-3-chlormethyl-2-hydroxy-3-methyl-1-(4-biphenylyloxy)1-(1,2,4-triazol-1-yl)-butan
4-Chlor-3-chlormethyl-2-hydroxy-3-methyl-1-(2-biphenylyloxy)-1-(1,2,4-triazol-1-yl)-butan
4-Chlor-3-chlormethyl-2-hydroxy-3-methyl-1-(4'-chlor-4-biphenylyloxy)-1-(1,2,4-triazol-1-yl)-butan
4-Chlor-3-chlormethyl-2-hydroxy-3-methyl-1-(4-fluorphenoxy)-1-(1,2,4-triazol-1-yl)-butan
4-Chlor-3-chlormethyl-2-hydroxy-3-methyl-1-(4-chlor-2-methylphenoxy)-1-(1,2,4-triazol-1-yl)-butan

Die außerdem als Ausgangsstoffe zu verwendenden sulfenylierten Carbamoylfluoride sind durch die Formel (III) allgemein definiert. Die Verbindungen der Formel (III) sind bekannt (vgl. DE-C-1 297 095 bzw. US-A-3 639 471) oder lassen sich nach allgemein üblichen und bekannten Verfahren herstellen. Man erhält sie z.B. durch Umsetzung der entsprechenden Carbamidsäurefluoride mit den entsprechenden Sulfenchloriden.

Als Ausgangsstoffe der Formel (III) seien beispielsweise genannt :

N-Methyl-N-trichlormethylmercapto-carbamoylfluorid
N-Methyl-N-dichlorfluormethylmercapto-carbamoylfluorid
N-Methyl-N-chlordifluormethylmercapto-carbamoylfluorid
N-Methyl-N-trifluormethylmercapto-carbamoyfluorid
N-Phenyl-N-trifluormethylmercapto-carbamoylfluorid
N-Phenyl-N-chlordifluormethylmercapto-carbamoylfluorid
N-Phenyl-N-dichlorfluormethylmercapto-carbamoylfluorid
N-Phenyl-N-trichlormethylmercapto-carbamoylfluorid.

Für die erfindungsgemäße Umsetzung kommen als Verdünnungsmittel inerte organische Lösungsmittel infrage. Hierzu gehören vorzugsweise Aether, wie Tetrahydrofuran und Dioxan ; Benzol ; in einzelnen Fällen auch chlorierte Kohlenwasserstoffe, wie Chloroform, Methylenchlorid oder Tetrachlorkohlenstoff.

Die Reaktionstemperaturen können in einem größeren Bereich variiert werden. Im allgemeinen arbeitet man zwischen 20 und 150 °C, vorzugsweise bei der Siedetemperatur des Lösungsmittels, beispielsweise zwischen 60 und 100 °C.

Bei der Durchführung des erfindungsgemäßen Verfahrens arbeitet man vorzugsweise in molaren Mengen. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt, gegebenenfalls wird ein Salz oder ein Metallsalz-Komplex hergestellt.

In einer bevorzugten Ausführungsform wird zweckmäßigerweise so verfahren, daß man von einem 2-Hydroxy-3,3-dimethyl-1-phenoxy-1-(1,2,4-triazol-1-yl)-butan-Derivat ausgeht, letzteres in einem geeigneten inerten organischen Lösungsmittel mittels Alkalimetall-hydrid oder Alkalimetall-amid in das Alkalimetall-alkoholat der Formel (II) überführt, und letzteres ohne Isolierung sofort mit einem Carbamoylfluorid der Formel (III) umsetzt, wobei unter Austritt von Alkalifluoriden die erfindungsgemäßen Verbindungen der Formel (I) in einem Arbeitsgang erhalten werden.

Nach einer weiteren bevorzugten Ausführungsform wird die Umsetzung des 2-Hydroxy-3,3-dimethyl-1-phenoxy-1-(1,2,4-triazol-1-yl)-butan-Derivats mit dem carbamoylfluorid der Formel (III) direkt in Gegenwart von Kaliumfluorid oder 4-Dimethylamino-pyridin als Base durchgeführt, wobei im Fall des Pyridins als Base, diese zum Reaktionsgemisch zugetropft und nicht von vornherein vorgelegt wird.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV.-Haupt- und der I. und II. sowie IV. bis VIII. Nebengruppe infrage, wobei Kupfer, Zink, Mangen, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z.B. durch Lösen des Metallsalzes in Alkohol, z.B. Aethanol, und Hinzufügen zur Verbindung der Formel (I). Mann kann Metallsalzkomplexe in bekannter Weise, z.B. durch Abfiltrieren, Isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen ; so zur Bekämpfung von Podosphaera-Arten, wie z.B. gegen den Erreger des Apfelmehltaus (Podosphaera leucotricha), von Erysiphe-Arten, wie z.B. gegen den Erreger des Gurkenmehltaus (Erysiphe cichoracearum) oder des Gerstenmehltaus bzw. des Getreidemehltaus (Erysiphe graminis) ; sowie zur Bekämpfung anderer Getreidekrankheiten, wie Getreiderost. Besonders hervorzuheben ist, daß die erfindungsgemäßen Wirkstoffe nicht nur eine protektive Wirkung entfalten, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

Mit nachfolgenden Beispielen werden einige Verwendungsmöglichkeiten eingehender dargestellt :

## Beispiel A

Sproßbehandlungs-Test/Getreidemehltau/protektiv (blattzerstörende Mykose).

Zur Herstellung einer zweckmäßigen Wirkstoffzubereitung nimmt man 0,25 gewichtsteile Wirkstoff in 25 Gewichtsteilen Dimethylformamid und 0,06 Gewichtsteilen Emulgator (Alkyl-aryl-polyglykoläther) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenjungpflanzen mit Sporen von Erysiphe graminis var. hordei.

Nach 6 Tagen Verweilzeit der Pflanzen bei einer Temperatur von 21-22 °C und einer Luftfeuchtigkeit von 80-90 % wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Mehltaubefall ist. Wirkstoffe, Wirkstoffkonzentrationen in der Spritzbrühe und Befallsgrad werden ermittelt. Bei diesem Test zeigen z.B. folgende Verbindungen eine sehr gute Wirkung, die derjenigen der aus dem Stand der Technik bekannten Verbindungen überlegen ist : Verbindungen gemäß Herstellungsbeispielen 2, 3, 4 und 1.

## Beispiel B

Gerstenmehltau-Test (Erysiphe greminis var. hordei)/systemisch (pilzliche Getreidesproßkrankheit).

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einem Volumteil Fruhstorfer Einheitserde und einem Volumteil Quarzsand ein. Die Keimung und der

Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21-22 °C und 80-90 % rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0 % keinen Befall und 100 % den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall werden ermittelt. Bei diesem Test zeigen z.B. folgende Verbindungen eine sehr gute Wirkung, die derjenigen der aus dem Stand der Technik bekannten Verbindungen überlegen ist :

Verbindungen gemäß Herstellungsbeispielen : 2, 4 und 1.

## Beispiel C

Sproßbehandlungs-Test/Getreiderost/protektiv (blattzerstörende Mykose).

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Alkyl-aryl-polyglykoläther auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1 %igem Wasseragar. Nach Antrocknen der Sporensuspension besprüht man die Weizenpflanzen mit der Wirkstoffzubereitung taufeucht und stellt sie zur Inkubation für 24 Stunden bei etwa 20 °C und einer 100 %igen Luftfeuchtigkeit in ein Gewächshaus.

Nach 10 Tagen Verweilzeit der Pflanzen bei einer Temperatur von 20 °C und einer Luftfeuchtigkeit von 80-90 % wertet man den Besatz der Pflanzen mit Rostpusteln aus. Der Befallsgrad wird in Prozenten des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Rostbefall ist.

Wirkstoffe, Wirkstoffkonzentrationen in der Spritzbrühe und Befallsgrade werden ermittelt. In diesem Test zeigen z.B. folgende Verbindungen eine sehr gute Wirkung, die derjenigen der aus dem Stand der Technik bekannten Verbindungen überlegen ist :

Verbindungen gemäß Herstellungsbeispielen 2, 3, 4 und 1.

## Beispiel D

Erysiphe-test (Gurken)/protektiv.

Lösungsmittel : 4,7 Gewichtsteile Aceton.

Emulgator : 0,3 Gewichtsteile Alkyl-aryl-polyglykoläther.

Wasser : 95,0 Gewichtsteile.

Man vermischt die für die gewünschte Wirkstoffkonzentration in der Spritzflüssigkeit nötige Wirkstoffmenge mit der angegebenen Menge Lösungsmittel und verdünnt das Konzentrat mit der an gegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23-24 °C und bei einer relativen Luftfeuchtigkeit von ca. 75 % im Gewächshaus aufgestellt.

Nach 12 Tagen wird der Befall der Gurkenpflanzen bestimmt. Die erhaltenen Boniturwerte werden in Prozent Befall umgerechnet. 0 % bedeutet keinen Befall, 100 % bedeutet, daß die Pflanzen vollständig befallen sind. Wirkstoffe, Wirkstoffkonzentrationen und Ergebnisse werden ermittelt. Bei diesem Test zeigen z.B. folgende Verbindungen eine sehr gute Wirkung, die derjenigen der aus dem Stand der Technik bekannten Verbindungen überlegen ist : Verbindungen gemäß Herstellungsbeispielen 2 und 3.

## Beispiel E

Podosphaera-Test (Apfel)/protektiv.

Lösungsmittel : 4,7 Gewichtsteile Aceton.

Emulgator : 0,3 Gewichtsteile Alkyl-aryl-polyglykoläther.

Wasser : 95,0 Gewichtsteile.

Man vermischt die für die gewünschte Wirkstoffkonzentration in der Spritzflüssigkeit nötige Wirkstoffmenge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium

befinden, bis zur Tropfnässe. Die Pflanzen verbleiben 24 Stunden bei 20 °C und einer relativen Luftfeuchtigkeit von 70 % im Gewächshaus. Anschließend werden die durch Bestäuben mit Konidien des Apfelmehltauerregers (Podosphaera leucotricha) inokuliert und in ein Gewächshaus mit einer Temperatur von 21 bis 23 °C und einer relativen Luftfeuchtigkeit von ca. 70 % gebracht.

10 Tage nach der Inokulation wird der Befall der Sämlinge bestimmt. Die erhaltenen Boniturwerte werden in Prozent Befall umgerechnet. 0 % bedeutet keinen Befall, 100 % bedeutet, daß die Pflanzen vollständig befallen sind.

Wirkstoffe, Wirkstoffkonzentrationen und Ergebnisse werden ermittelt. Bei diesem Test zeigen z.B. folgende Verbindungen eine sehr gute Wirkung, die derjenigen der aus dem Stand der Technik bekannten Verbindungen überlegen ist : Verbindungen gemäß Herstellungsbeispielen 3 und 1.

## Herstellungsbeispiele

### Beispiel 1

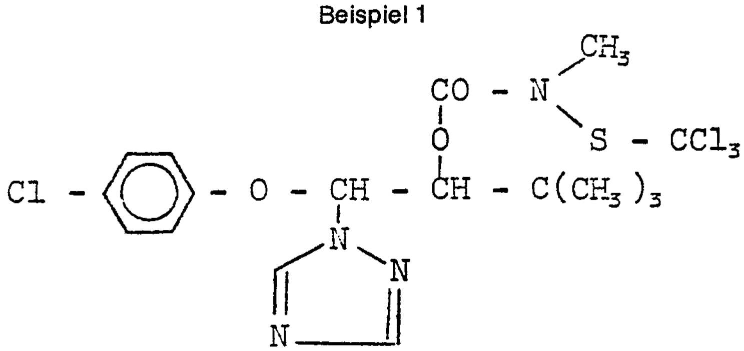

22,3 g (0,075 Mol) 1-(4-Chlorphenoxy)-3,3-dimethyl-1-(1,2,4-triazol-1-yl)-butan-2-ol als reines Diastereomeres werden in 200 ml Tetrahydrofuran gelöst und unter Rühren bei Raumtemperatur zu 2,3 g 80 %-igem Natriumhydrid in 150 ml Tetrahydrofurangetropft. Danach läßt man 12 Stunden bei 50 °C rühren. Nach dem Abkühlen werden bei Raumtemperatur zu dem so erhaltenen Natriumsalz 17 g (0,075 Mol) N-Methyl-N-trichlormethylmercapto-carbamoylfluorid zugetropft, wobei die Temperatur auf ca. 35 °C ansteigt. Anschließend erhitzt man 16 Stunden unter Rückfluß, läßt abkühlen und engt durch Abdestillieren des Lösungsmittels ein. Der ölige Rückstand wird in 500 ml Methylenchlorid aufgenommen, zweimal mit je 1 000 ml Wasser gewaschen, über Natriumsulfat getrocknet und eingeengt. Das zurückbleibende Oel wird in 300 ml Isopropyläther aufgenommen, wobei es auskristallisiert. Man erhält 14,5 g (37 % der Theorie) 1-(4-Chlorphenoxy)-3,3-dimethyl-2-(N-methyl-N-trichlormethylmercapto-carbamoyloxy)-1-(1,2,4-triazol-1-yl)-butan als reines Diastereomeres vom Schmelzpunkt 110-112 °C.

Analog werden die nachfolgenden Beispiele der allgemeinen Formel

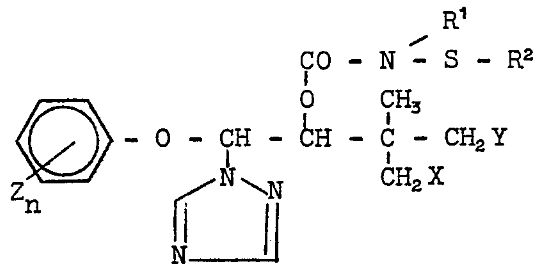

(I)

erhalten:

| Bsp. Nr. | $R^1$ | $R^2$ | X | Y | Zn | Schmelzpunkt (°C) |
|---|---|---|---|---|---|---|
| 2 | $CH_3$ | $—CCl_3$ | H | H | 4— 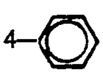 | 127-29 (A-Form) |
| 3 | $CH_3$ | $—CCl_2F$ | H | H | 4— 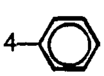 | 99-101 (A-Form) |
| 4 | $CH_3$ | $—CCl_2F$ | H | H | 4—Cl | 84-89 (A-Form) |
| 5 | $CH_3$ | $—CCl_3$ | H | H | 4—Br | 112 |

erhalten:

| Bsp. Nr. | $R^1$ | $R^2$ | X | Y | Zn | Schmelzpunkt (°C) |
|---|---|---|---|---|---|---|
| 6 | 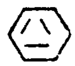 | $-CCl_2F$ | H | H | 4—Br | 211 (× 1/2 NDS) (A-Form) |
| 7 | 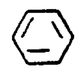 | $-CCl_2F$ | H | H | 2,4—$Cl_2$ | 250 (× 1/2 NDS) (A-Form) |

NDS = Naphthalindisulfonsäure
A- und B-Form = jeweils eine der beiden möglichen geometrischen Isomeren

**Ansprüche**

1. Sulfenylierte Carbamoyl-triazolyl-O,N-acetale der allgemeinen Formel I

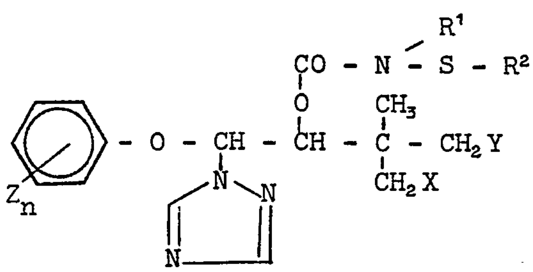

(I)

in welcher
$R^1$ für Methyl oder gegebenenfalls durch Chlor oder Methyl substituiertes Phenyl steht,
$R^2$ für Trifluormethyl, Chlordifluormethyl, Dichlorfluormethyl und Trichlormethyl steht,
X und Y für Wasserstoff, Fluor, Chlor oder Brom stehen,
Z für Fluor, Chlor, Brom, Methyl, Äthyl, sowie für gegebenenfalls durch Chlor oder Methyl substituiertes Phenyl steht, und
n für die Zahlen 0, 1 oder 2 steht,
und deren physiologisch verträglichen Säureadditions-Salze und Metallsalz-Komplexe.

2. Verfahren zur Herstellung der sulfenylierten Carbamoyl-triazolyl-O,N-acetale nach Anspruch 1, dadurch gekennzeichnet, daß man ein Alkoholat eines 2-Hydroxy-1-phenoxy-1-triazolyl-butans der Formel II

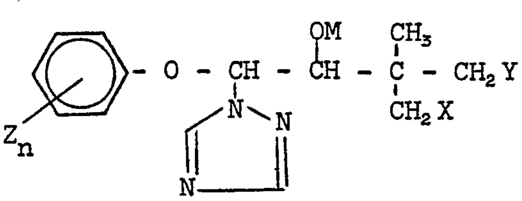

(II)

in welcher
X, Y, Z und n die in Anspruch 1 angegebene Bedeutung haben und
M für ein Alkalimetall, eine quarternäre Ammonium- oder Phosphonium-Gruppe steht,
mit einem sulfenylierten Carbamoylfluorid der Formel III

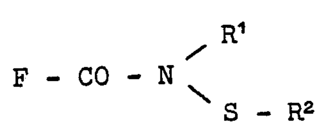

(III)

in welcher
$R^1$ und $R^2$ die in Anspruch 1 angegebene Bedeutung haben,
in Gegenwart eines Verdünnungsmittels umsetzt, und weiterhin gegebenenfalls noch die erhaltenen

sulfenylierten Carbamoyl-triazolyl-O,N-acetale der Formel (I) durch Umsetzung mit Säuren in deren Salze oder durch Reaktion mit Metallsalzen in die entsprechenden Metallsalz-Komplexe überführt.

3. Fungizide Mittel, gekennzeichnet durch einen Gehalt an mindestens einem sulfenylierten Carbamoyl-triazolyl-O,N-acetal gemäß Anspruch 1.

4. Verwendung von sulfenylierten Carbamoyl-triazolyl-O,N-acetalen gemäß Anspruch 1 zur Bekämpfung von Pilzen.

**Claims**

1. Sulphenylated carbamoyl-triazolyl-O,N-acetals of the general formula I

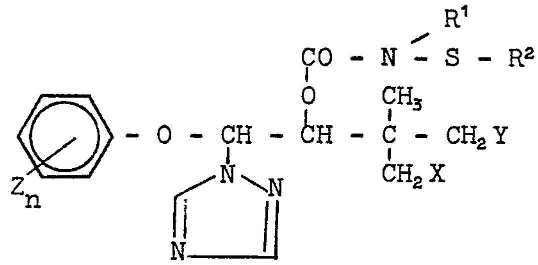

(I)

in which

$R^1$ represents methyl or phenyl optionally substituted by chlorine or methyl,

$R^2$ represents trifluoromethyl, chlorodifluoromethyl, dichlorofluoromethyl and trichloromethyl,

X and Y represent hydrogen, fluorine, chlorine or bromine,

Z represents fluorine, chlorine, bromine, methyl, ethyl, and phenyl optionally substituted by chlorine or methyl, and

n represents the numbers 0, 1 or 2,

and physiologically acceptable acid addition salts and metal salt complexes thereof.

2. Process for the preparation of the sulphenylated carbamoyl-triazolyl-O,N-acetals according to claim 1, characterised in that an alcoholate of a 2-hydroxy-1-phenoxy-1-triazolyl-butane of the formula II

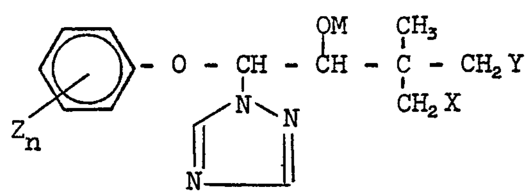

(II)

in which

X, Y, Z and n have the meaning given in claim 1 and

M represents an alkali metal or a quaternary ammonium or phosphonium group,

is reacted with a sulphenylated carbamoyl fluoride of the formula III

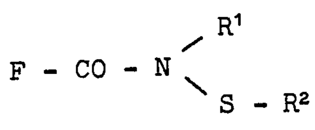

(III)

in which

$R^1$ and $R^2$ have the meaning given in claim 1,

in the presence of a diluent and the resulting sulphenylated carbamoyl-triazolyl-O,N-acetals of the formula (I) are optionally further converted into the salts thereof by reaction with acids or, by reaction with metal salts, into the corresponding metal salt complexes.

3. Fungicidal compositions, characterised in that they contain at least one sulphenylated carbamoyl-triazolyl-O,N-acetal according to claim 1.

4. Use of sulphenylated carbamoyl-triazolyl-O,N-acetals according to claim 1 for combating fungi.

**Revendications**

1. Carbamoyl-triazolyl-O,N-acétals sulfénylés de formule générale I

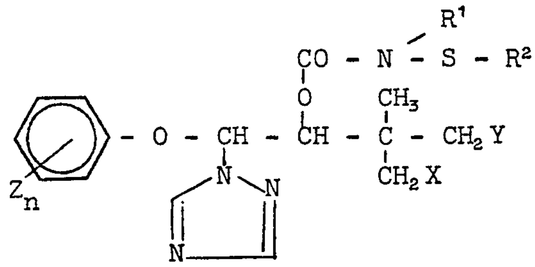

(I)

dans laquelle

$R^1$ représente un méthyl ou un phényle éventuellement substitué par du chlore ou méthyle,

$R^2$ représente trifluorométhyle, chlorodifluorométhyle, dichlorofluorométhyle et trichlorométhyle,

X et Y représentent de l'hydrogène, du fluor, du chlore ou du brome,

Z représente du fluor, chlore, brome, méthyle, éthyle de même qu'un phényle éventuellement substitué par du chlore ou méthyle et

n représente les nombres 0, 1 ou 2,

leurs sels d'addition d'acides physiologiquement compatibles et complexes de sels métalliques.

2. Procédé de fabrication des carbamoyl-triazolyl-O,N-acétals sulfénylés selon la revendication 1, caractérisé en ce qu'on fait réagir un alcoolate d'un 2-hydroxy-1-phénoxy-1-triazolyl-butane de formule II

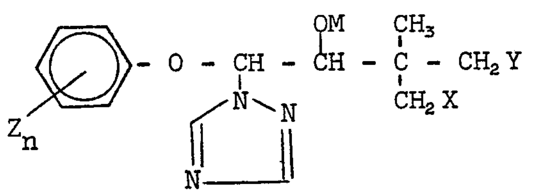

(II)

dans laquelle

X, Y, Z et n ont la signification indiquée à la revendication 1 et

M représente un métal alcalin, un groupe ammonium ou phosphonium quaternaire,

avec un fluorure de carbamoyle sulfénylé de formule III

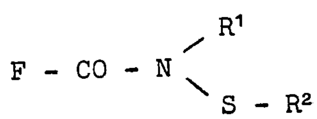

(III)

dans laquelle

$R^1$ et $R^2$ ont la signification indiquée à la revendication 1,

en présence d'un diluant, et en ce qu'en outre, éventuellement, on convertit les carbamoyl-triazolyl-O,N-acétals sulfénylés obtenus de formule (I), par réaction avec des acides, en leurs sels, ou, par réaction avec des sels métalliques, en les complexes de sels métalliques correspondants.

3. Agents fongicides, caractérisés par une teneur en au moins un carbamoyl-triazolyl-O,N-acétal sulfénylé selon la revendication 1.

4. Utilisation des carbamoyl-triazolyl-O,N-acétals sulfénylés selon la revendication 1 pour combattre les champignons.